# EUROPEAN PATENT APPLICATION

(11) **EP 0 967 276 A2**
(43) Date of publication of application: **29.12.1999**
(21) Application number: 99304935.2
(22) Date of filing: 23.06.1999
(51) Int. Cl.: C12N 15/12, C07K 14/46, A61K 38/17, C12N 15/85

(54) **Anti-tumor agent comprising salmosin**

(30) Priority: 23.06.1998 KR 9823778; 04.06.1999 KR 9920579
(71) Applicant: Kim, Doo-Sik, Seodaemun-gu, Seoul 120-110 (KR)
(72) Inventor: Kim, Doo-Sik, Seodaemun-gu, Seoul 120-110 (KR); Chung, Kwang Hoe, Bundang-gu, Sungnam, Kyounggi-do 463-070 (KR); Kang, In-Cheol, Paldal-gu, Suwon, Kyounggi-do, 442-374 (KR)
(74) Representative: Woods, Geoffrey Corlett

(57) **Abstract**

The present invention relates to a Salmosin polypeptide and homologues and fragments thereof for use in a method of treatment of the human or animal body, in particular for anti-tumor therapy.

## Description

### Field of the Invention

The present invention relates to Salmosin for use as an anti-tumor agent. The invention also relates to polynucleotides encoding Salmosin and to the use of such polynucleotides in the treatment of tumors.

### Background of the Invention

Tumor invasion and metastasis are the biological phenomena in which cancer cells lethally spread throughout the body. First, cancer cells detach from the primary site (e.g., epithelial tissue) and cross the basement membrane separating them from other tissue layers. Some of these invasive cells penetrate the basement membrane and endothelial cells surrounding a blood vessel and circulate via the bloodstream. The circulating cancer cells may adhere to and penetrate a capillary wall to create a secondary tumor. Perhaps, fewer than one in 10,000 cancer cells that escape the primary site survives to form a colony in another tissue (see: Erkki Ruoslahti, Scientific American, 72-77, Sep., 1996).

Therefore, tumor metastasis and invasion require adhesive interaction between cells and extracellular matrix ("ECM"). In the course of tumor metastasis, tumor cells can cause endothelial cells to retract, exposing the subendothelial basement membrane and allowing the tumor cells to adhere efficiently to ECM proteins of the surrounding stroma (see: Hynes, R. O., Cell, 48:549, 1987). These matrix proteins promote cells adhesion by binding to specific cell surface receptors, including a member of integrin family.

In terms of structure, each integrin is a heterodimer consisting of α and β subunits which are noncovalently associated with each other. The β1 subfamily is a primary mediator of extracellular matrix adhesions. It has been reported that β1 integrins may have other functions, such as to mediate cell-cell adhesion directly (see: Larjava, H., et al,. J. Cell. Biol., 110:803-815, 1990). Integrins of the β2 subfamily are found on leukocytes where they mediate cell-cell interactions. The β3 subfamily, which includes the platelet glycoprotein IIb/IIIa complex and the vitronectin receptor, may play an important role in the development of tumor invasiveness and malignancy (see: Albelda, S. M., et al., Cancer Res., 50:6757-6764, 1990).

The integrin receptor complex that spans the plasma membrane links the integral cytoskeletal network of a cell with the extracellular environment. Common or characteristic core sequences in cell adhesion molecules such as fibrinogen, vitronectin and laminin have been considered to contribute to cell adhesion and to the spread or integration of cells.

It has been suggested that tumorigenesis and metastasis are closely associated with the biological role of integrins (see: Giancotti, F. G. and Rouslahti, E., Cell, 60:849-859, 1990: Hynes, R. O., Cell, 69:11-25, 1992; Nip, J., et al., J. Clin. Invest., 96:2096-2103, 1995).

It has been reported that overexpression of the fibronectin receptor, α5β1, suppresses the transformed phenotype of Chinese hamster ovary cells, that integrin α5β1 is reduced in ras-transformed rodent cells (see: Plantefaben, L. C. and Hynes, R. O., Cell, 56:281-290, 1989) and that superfibronectin, a polymeric fibrillar form of fibronectin, prevents tumor metastasis and tumor formation (see: Pasqualini, R., et al., Nature Medicine, 2:1197-1203, 1996).

Integrin αvβ3 is a specific marker of the most malignant cells, suggesting a crucial role of this adhesion receptor in the malignant growth of human melanoma (see: Albelda, S. M.. Et al., Cancer Res., 50:6757-6764, 1990). Integrin αvβ3 gene expression and the resulting adhesive phenotype are directly involved in the proliferation of human melanoma in vivo (see: Felding-Habermann, J. Clin. Invest., 89:2018-2022, 1992).

Angiogenesis is a biological process of forming new blood vessels as outgrowths from preexisting blood vessels (see: Folkman, J. and D'Amore, P. A, Cell, 87:1153-1155, 1996). This process plays a key role in the progression of a solid tumor, as well as in normal development, wound healing and inflammation and vascular cell adhesion molecules in smooth muscle and endothelial cells contribute to its regulation (see: Nguyen, M., et al., Nature, 365-267, 1993).

The switch of angiogenic phenotype of a tumor may be caused by losing balance between positive and negative modulators involved in neovascularization. Recently, it has been reported that two cytokine-dependent pathways of angiogenesis were shown to exist and were defined by distinct vascular cell integrins, αvβ3 and αvβ5 that become expressed on angiogenic vascular cells where they play a critical role in angiogenesis induced by basic fibroblast growth factor ("bFGF"), tumor necrosis factor-alpha (TNF-α), vascular endothelial growth factor (VEGF), and fragments of human tumors (see: Friedlander, M., et al., Science, 270:1500-502, 1995). Activation of αvβ3 integrin stimulates a survival signal that facilitates blood vessel growth and differentiation indicating that signalling events by both cytokine and integrin receptors are closely associated with the growth of new blood vessels (see: Brooks. P. C. et al., Cell, 79:1157-1164, 1994).

Several endogenous angiogenic inhibitors have been identified as following: interferon-α, -γ (see: Friesel, R., et al., J. Cell. Biol., 104:689-696, 1987; Ezekowitz, R. A., et al., N. Engl. J. Med., 324:1456-1463, 1992); interferon-inducible protein 10 (see: Angiolillo, A. L., et al., J. Exp. Med., 182:155-162, 1995; Strieter, R. M., et al., Biochem. Biophys. Res. Comm., 210:51-57, 1995); angiostatin and endostatin that specifically suppress endothelial cell proliferation (see: O'Reilly, M. S., et al., Cell, 79:315-328, 1994; O'Reilly M. S., et al., Cell, 88:277-285, 1997); gro-β (see: Cao, Y., et al., J. Exp. Med., 182:2069-2077, 1995); the 16kDa N-terminal fragment of prolactin (see: Clapp, C., et al., Endocrinology, 133:1292-1299, 1993); and, platelet factor-4 (see: Maione, T., et al., Science, 247:77-79, 1990; Gupta, S. K., et al., Proc. Natl. Acad. Sci., USA, 92:7799-7803, 1995).

It has been well known that disintegrins are a family of small proteins mainly derived from snake venom (see: Niewiarowski, S., et al., Semin. Hematol., 31:289-300, 1994). Most of the disintegrins contain Arg-Gly-Asp (RGD) or Lys-Gly-Asp (KGD) sequence which is the structural motif recognized by a platelet fibrogen receptor α2bβ3. Disintegrins also act as potent antagonists of several integrins including αvβ3 and α5β1. There are several reports demonstrating that disintegrins inhibit tumor metastasis by blocking tumor cell adhesion to ECM (see: Trika, M., et al., Cancer Res., 54 (8):4993-4998, 1994).

Integrin αvβ3 was identified as a marker of angiogenic blood vessels in chick embryo and human (see: Brooks. P. C., et al., Science, 264:569-571, 1994). Monoclonal antibody against αvβ3 was able to perturb angiogenesis by inducing apoptosis in the endothelial cells of the newly formed blood vessels. Application of synthetic peptides containing the RGD sequence that inhibit ligand binding to integrin αvβ3 suppressed tumor-induced angiogenesis on chick chorioallantoic membrane ("CAM") (see: Brooks. P. C., et al., Cell, 99:1157-1164, 1994), and also suppressed the function of angiogenin which assists adhesion and diffusion of endothelial cells. Recently, triflavin, a disintegrin derived from snake venom, is reported to inhibit angiogenesis induced by TNF-α.

The present inventors isolated Salmosin derived from venom of Korean snake, *Agkistrodon halys brevicaudus,* and characterized that: it is a novel protein of 7.5kDa having a strong inhibitory activity against platelet agglutination (see: Korean Patent No. 142606, SEQ ID NO: 1), and it contains RGD sequence which is known to inhibit ligand binding to integrin αvβ3.

The present invention relates to an anti-tumor agent comprising a disintegrin derived from snake venom, more specifically, to an anti-tumor agent comprising Salmosin which is a novel disintegrin containing Arg-Gly-Asp (RGD) sequence and derived from venom of Korean snake, *Agkistrodon halys brevicaudus,* as an active ingredient.

### Summary of the Invention

Here we report the finding that Salmosin can strongly inhibit tumor angiogenesis which is essential to cancer cell growth and metastasis without affecting proliferation of normal endothelial cells. We also report the isolation and purification of Salmosin from the venom of Korean snake *Agkistrodon halys brevicaudus,* the cloning of the cDNA encoding Salmosin and the overexpression of recombinant Salmosin in *E.coli.*

Accordingly, the invention provides:
- a polypeptide which comprises the sequence of SEQ ID NO: 1, a polypeptide substantially homologous thereto, or a fragment of the polypeptides of, or homologue of SEQ ID NO: 1 for use in a method of treatment of the human or animal body.
- use of a polypeptide of the invention in the manufacture of a medicament for anti-tumor therapy.
- use of a polypeptide of the invention in the manufacture of a medicament for the inhibition or reduction of tumor cell angiogenesis.
- use of a polypeptide of the invention in the manufacture of a medicament for the inhibition of metastatic tumor formation.
- use of a polypeptide of the invention in the manufacture of a medicament for the inhibition or reduction of metastatic tumor growth.
- use of a polypeptide of the invention in the manufacture of a medicament for the treatment of skin cancer, laryngeal cancer, uterine cancer, colon cancer, lung cancer or bone marrow cancer.
- a polynucleotide selected from:
   (a) a polynucleotide comprising the nucleotide sequence of SEQ ID NO: 3 or the complement thereof
   (b) a polynucleotide comprising a nucleotide sequence capable of hybridizing to the nucleotide sequence defined in (a)
   (c) a polynucleotide comprising a nucleotide sequence which is degenerate as a result of the genetic code to the polynucleotides defined in (a) and (b)
   (d) a polynucleotide which encodes a polypeptide of the invention.
- an expression vector comprising a polynucleotide of the invention operably linked to regulatory sequences capable of directing expression of said polynucleotide in a host cell.
- a pharmaceutical composition comprising a polypeptide, polynucleotide or vector of the invention and a pharmaceutically acceptable carrier.
- a polynucleotide or vector of the invention for use in a method of treatment of the human or animal body, for example in anti-tumor therapy.

### Brief Description of Drawings

The objects and features of the present invention will become apparent from the following description given in conjunction with the accompanying drawings, in which:

Figure 1 is a genetic map of a recombinant expression vector ApMA-PRK153 encoding phosphoribulose kinase (PRK).

Figure 2A is a genetic map of a recombinant expression vector ΔpMASIN1 encoding a fusion protein which consists of PRK153, urokinase cleavage site and Salmosin.

Figure 2B is a photograph of SDS-PAGE pattern showing recombinant Salmosin and isolated Salmosin.

Figure 3 shows photographs of mouse lung in which B16 melanoma cell metastasis is suppressed by Salmosin.

Figure 4A is a graph showing the suppressive effect of Salmosin on proliferation of BCE (bovine capillary endothelial) cell stimulated by bFGF (basic fibroblast growth factor).

Figure 4B is a graph showing the suppressive effect of anti-αvβ3 monoclonal antibody and Salmosin on proliferation of BCE cells stimulated by bFGF.

Figure 5A is a photograph showing CAM (chick choriollantoic membrane) angiogenesis induced by bFGF.

Figure 5B is a photograph showing the suppressive effect of anti-αvβ3 monoclonal antibody on CAM angiogenesis induced by bFGF.

Figure 5C is a photograph showing the suppressive effect of Salmosin on CAM angiogenesis induced by bFGF.

Figure 6A is a graph showing the suppressive effect of Salmosin or other antagonists against αvβ3 integrin (i.e. anti-αvβ3 antibody or GRGDSP) on adhesion of vitronectin and BCE cell.

Figure 6B is a graph showing the suppressive effect of Salmosin or other antagonists against αvβ3 integrin (i.e. anti-αvβ3 antibody or GRGDSP) on adhesion of Salmosin and BCE cell.

Figure 7 shows photographs of mouse lungs showing the suppressive effect of Salmosin on growth of metastatic Lewis lung carcinoma (top: PBS treatment, bottom: Salmosin treatment).

Figure 8 is a graph showing the suppressive effect of Salmosin on growth of solid Lewis lung carcinoma.

### Detailed Description of the Invention

The present inventors have isolated and purified Salmosin from venom of Korean snake, *Agkistrodon halys brevicaudus,* screened cDNA thereof and overexpressed recombinant Salmosin in *E.coli.* They have investigated the biological effect of both isolated and recombinant Salmosin on tumor metastasis and tumor growth both *in vivo* and *in vitro.* As a result, they found that: Salmosin strongly inhibits the function of αvβ3 integrin which is crucial to tumor angiogenesis; and, it effectively suppresses metastasis and growth of tumors.

The present invention is further illustrated as follows.

Salmosin was isolated and purified from venom of Korean snake, *Agkistrodon halys brevicaudus,* using a series of column chromatographies and the cDNA encoding Salmosin was cloned from the venom gland cDNA library of *Agkistrodon halys brevicaudus.* To investigate whether Salmosin may suppress metastasis of tumor cells, Salmosin was mixed with melanoma cells and injected into the lateral tail vein of mouse. Salmosin did not inhibit the proliferation of tumor cell itself in vitro, while it inhibited tumor cell angiogenesis in a dose-dependent manner. These findings strongly suggested that the suppressive effect of Salmosin on tumor metastasis is grounded on inhibition of the function of integrin relating to tumor metastasis, not on its cytotoxicity. In this connection, the present inventors investigated whether Salmosin may inhibit the function of αvβ3 integrin which is associated with tumor angiogenesis using a BCE cell proliferation test. As a result, it was found that: Salmosin inhibits tumor-induced angiogenesis without affecting the preexisting blood vessels or normal angiogenesis; and, the suppressive effect of Salmosin on BCE cell proliferation results from the direct binding of Salmosin to the vitronectin receptor, αvβ3 integrin, on the surface of BCE cells.

It was known that disintegrins can inhibit colony formation of a metastatic tumor, but is was not clear whether disintegrins can inhibit the growth of a metastatic tumor that has already formed. Thus, to examine the effect of Salmosin on the growth of metastatic tumors, metastatic Lewis lung carcinoma cells were injected to the mouse and after colony formation, Salmosin was injected. As a result, it was demonstrated that metastatic tumor growth was effectively suppressed without any symptom of cytotoxicity and Salmosin also inhibited the growth of a solid tumor.

### A. Polypeptides

Polypeptides of the invention or for use in the invention are the Salmosin polypeptide which comprises the sequences set out in SEQ ID NO: 1, a homologue thereof or a fragment of Salmosin or a Salmosin homologue.

Polypeptides provided by the invention include variants of SEQ ID NO: 1, including naturally occurring allelic variants and synthetic variants which are substantially homologous to said polypeptides and contain RGD or KGD at positions homologous to amino acids 51 to 53 of SEQ ID No. 1. In this context, substantial homology is regarded as a sequence which has at least 70%, e.g. 80% or 90% amino acid homology (identity) over 30 amino acids with the sequence of SEQ ID No. 1.

Polypeptides also include other Salmosin homologues, and variants thereof as defined above, from other species including animals such as snakes.

Polypeptides of the invention also include fragments of the above mentioned full length polypeptides and variants thereof, including fragments of the sequences set out in SEQ ID No. 1. Preferred fragments include those which include the RGD tripeptide. Suitable fragments will be at least about 5, e.g. 10, 12, 15 or 20 amino acids in size. Polypeptide fragments of the Salmosin proteins and allelic and species variants thereof may contain one or more (e.g. 2, 3, 5, or 10) substitutions, deletions or insertions, including conserved substitutions.

Conserved substitutions may be made according to the following table which indicates conservative substitutions, where amino acids on the same block in the second column and preferably in the same line in the third column may be substituted for each other:

| | | |
|---|---|---|
| ALIPHATIC | Non-polar | G A P |
| | | I L V |
| | Polar - uncharged | C S T M |
| | | N Q |
| | Polar - charged | D E |
| | | K R |
| AROMATIC | | H F W Y |
| OTHER | | N Q D E |

Polypeptides of the invention may be in a substantially isolated form. It will be understood that the polypeptide may be mixed with carriers or diluents which will not interfere with the intended purpose of the polypeptide and still be regarded as substantially isolated. A polypeptide of the invention may also be in a substantially purified form, in which case it will generally comprise the polypeptide in a preparation in which more than 90%, e.g. 95%, 98% or 99% of the polypeptide in the preparation is a polypeptide of the invention. Polypeptides of the invention may be modified for example by the addition of histidine residues to assist their purification or by the addition of a signal sequence to promote their secretion from a cell.

Polypeptides provided by the invention may be made by synthetic means (e.g. as described by Geysen et *al.,* 1996) or recombinantly, as described below. Alternatively, Salmosin may be isolated and purified from the venom of *Agkistrodon halys brevicaudus,* for example, by column chromatography.

Particularly preferred polypeptides of the invention include those spanning the RGD sequence represented as amino acids 51 to 53 in SEQ ID No. 1. Fragments as defined above from this region containing the RGD sequence are particularly preferred. The polypeptides and fragments thereof may contain amino acid alterations as defined above.

The polypeptides of the invention may be introduced into a cell by *in situ* expression of the polypeptide from a recombinant expression vector (see below). The expression vector optionally carries an inducible promoter to control the expression of the polypeptide. The use of mammalian host cells is expected to provide for such post-translational modifications (e.g. myristolation, glycosylation, truncation, lapidation and tyrosine, serine or threonine phosphorylation) as may be needed to confer optimal biological activity on recombinant expression products of the invention. Such cell culture systems in which polypeptide of the invention are expressed may be used in assay systems to identify candidate substances which interfere with or enhance the functions of the polypeptides of the invention in the cell.

### B. Polynucleotides

In the following description polynucleotides of the invention may comprise DNA or RNA. They may be single or double stranded. They may also be polynucleotides which include within them synthetic or modified nucleotides. A number of different types of modification to oligonucleotides are known in the art. These include methylphosphonate and phosphorothioate backbones, addition of acridine or polylysine chains at the 3' and/or 5' ends of the molecule. For the purposes of the present invention, it is to be understood that the polynucleotides described herein may be modified by any method available in the art. Such modifications may be carried out in order to enhance the *in vivo* activity or life span of polynucleotides of the invention.

Polynucleotides of the invention capable of selectively hybridising to the DNA of SEQ ID No. 3 will be generally at least 70%, preferably at least 80 or 90% and more preferably at least 95% homologous to the corresponding DNA of SEQ ID No. 3 over a region of at least 20, preferably at least 25 or 30, for instance at least 40, 60 or 100 or more contiguous nucleotides. Preferred polynucleotides of the invention will comprise regions encompassing the region encoding the RGD amino acid sequence of Salmosin at nucleotides 151 to 159 of SEQ ID No. 3. Such preferred polynucleotides will encode RGD or KGD at nucleotides 151 to 159 of SEQ ID No. 3.

It is to be understood that skilled persons may, using routine techniques, make nucleotide substitutions that do not affect the polypeptide sequence encoded by the polynucleotides of the invention to reflect the codon usage of any particular host organism in which the polypeptides of the invention are to be expressed.

Any combination of the above mentioned degrees of homology and minimum sizes may be used to define polynucleotides of the invention, with the more stringent combinations (i.e. higher homology over longer lengths) being preferred. Thus for example a polynucleotide which is at least 80% homologous over 25, preferably 30 nucleotides forms one aspect of the invention, as does a polynucleotide which is at least 90% homologous over 40 nucleotides.

Polynucleotides of the invention may be used to produce a primer, e.g. a PCR primer, a primer for an alternative amplification reaction, a probe e.g. labelled with a revealing label by conventional means using radioactive or non-radioactive labels, or the polynucleotides may be cloned into vectors. Such primers, probes and other fragments will be at least 15, preferably at least 20, for example at least 25, 30 or 40 nucleotides in length, and are also encompassed by the term polynucleotides of the invention as used herein.

Polynucleotides such as a DNA polynucleotide and primers according to the invention may be produced recombinantly, synthetically, or by any means available to those of skill in the art. They may also be cloned by standard techniques.

In general, primers will be produced by synthetic means, involving a step wise manufacture of the desired nucleic acid sequence one nucleotide at a time. Techniques for accomplishing this using automated techniques are readily available in the art.

Longer polynucleotides will generally be produced using recombinant means, for example using a PCR (polymerase chain reaction) cloning techniques. This will involve making a pair of primers (e.g. of about 15-30 nucleotides) to a region of the Salmosin gene which it is desired to clone, bringing the primers into contact with mRNA or cDNA obtained from an animal cell, performing a polymerase chain reaction under conditions which bring about amplification of the desired region, isolating the amplified fragment (e.g. by purifying the reaction mixture on an agarose gel) and recovering the amplified DNA. The primers may be designed to contain suitable restriction enzyme recognition sites so that the amplified DNA can be cloned into a suitable cloning vector.

Such techniques may be used to obtain all or part of the Salmosin sequence described herein. Genomic clones containing the Salmosin gene and its introns and promoter regions may also be obtained in an analogous manner, starting with genomic DNA from an animal cell.

Although in general the techniques mentioned herein are well known in the art, reference may be made in particular to Sambrook *et al.,* Molecular Cloning, A Laboratory Manual (1989) and Ausubel *et al.,* Current Protocols in Molecular Biology (1995), John Wiley & Sons, Inc.

Polynucleotides which are not 100% homologous to the sequences of the present invention but fall within the scope of the invention can be obtained in a number of ways. Other *Agkistrodon halys brevicaudus* allelic variants of the Salmosin sequence described herein may be obtained for example by probing genomic DNA libraries made from a range of individuals, for example individuals from different populations. In addition, other animal, particularly reptile (e.g. snake), homologues of Salmosin may be obtained and such homologues and fragments thereof in general will be capable of selectively hybridising to SEQ ID No. 3. Such sequences may be obtained by probing cDNA libraries made from dividing cells or tissues or genomic DNA libraries from other animal species, and probing such libraries with probes comprising all or part of SEQ ID. 3 under conditions of medium to high stringency (for example 0.03M sodium chloride and 0.03M sodium citrate at from about 50°C to about 60°C). Nucleic acid probes comprising all or part of SEQ ID No. 3 may be used to probe cDNA libraries, preferably from toxin glands, to obtain homologues of Salmosin.

Allelic variants and species homologues may also be obtained using degenerate PCR which will use primers designed to target sequences within the cDNA encoding Salmosin. The primers will contain one or more degenerate positions and will be used at stringency conditions lower than those used for cloning sequences with single sequence primers against known sequences. In particular, primers can be designed to target the RGD tripeptide encoding domains described above.

Alternatively, such polynucleotides may be obtained by site directed mutagenesis of the Salmosin sequence or allelic variants thereof. This may be useful where for example silent codon changes are required to sequences to optimise codon preferences for a particular host cell in which the polynucleotide sequences are being expressed. Other sequence changes may be desired in order to introduce restriction enzyme recognition sites, or to alter the property or function of the polypeptides encoded by the polynucleotides. For example, it may be desirable to introduce changes to enhance the integrin binding properties of Salmosin encoded by the polynucleotides.

The invention further provides double stranded polynucleotides comprising a polynucleotide of the invention and its complement.

Polynucleotides or primers of the invention may carry a revealing label. Suitable labels include radioisotopes such as ³²P or ³⁵S, enzyme labels, or other protein labels such as biotin. Such labels may be added to polynucleotides or primers of the invention and may be detected using by techniques known per se.

The present invention also provides polynucleotides encoding the polypeptides of the invention described below. Because such polynucleotides will be useful as sequences for recombinant production of polypeptides of the invention, it is not necessary for them to be selectively hybridisable to the sequence of SEQ ID No. 3 although this will generally be desirable. Otherwise, such polynucleotides may be labelled, used, and made as described above if desired. Polypeptides of the invention are described below.

### C. Vectors.

Polynucleotides of the invention can be incorporated into a recombinant replicable vector. The vector may be used to replicate the nucleic acid in a compatible host cell. Thus in a further embodiment, the invention provides a method of making polynucleotides of the invention by introducing a polynucleotide of the invention into a replicable vector, introducing the vector into a compatible host cell, and growing the host cell under conditions which bring about replication of the vector. The vector may be recovered from the host cell. Suitable host cells are described below in connection with expression vectors.

### Expression Vectors.

Preferably, a polynucleotide of the invention in a vector is operably linked to a regulatory sequence which is capable of providing for the expression of the coding sequence by the host cell, i.e. the vector is an expression vector. The term "operably linked" refers to a juxtaposition wherein the components described are in a relationship permitting them to function in their intended manner. A regulatory sequence "operably linked" to a coding sequence is ligated in such a way that expression of the coding sequence is achieved under condition compatible with the control sequences.

The term "regulatory sequences" includes promoters and enhancers and other expression regulation signals. These may be selected to be compatible with the host cell for which the expression vector is designed. For example, yeast regulatory sequences include *S. cerevisiae* GAL4 and ADH promoters, S. *pombe* nmt1 and adh promoters. Mammalian promoters, such as a-actin promoters, may be used. Mammalian promoters also include the metallothionein promoter which can upregulate expression in response to heavy metals such as cadmium and is thus an inducible promoter. Tissue-specific promoters, for example neuronal cell specific may be used. Viral promoters may also be used, for example the Moloney murine leukaemia virus long terminal repeat (MMLV LTR), the promoter rous sarcoma virus (RSV) LTR promoter, the SV40 promoter, the human cytomegalovirus (CMV) IE promoter, herpes simplex virus promoters or adenovirus promoters. All these promoters are readily available in the art.

Such vectors may be transformed into a suitable host cell as described above to provide for expression of a polypeptide of the invention. Thus, in a further aspect the invention provides a process for preparing polypeptides according to the invention which comprises cultivating a host cell transformed or transfected with an expression vector as described above under conditions to provide for expression by the vector of a coding sequence encoding the polypeptides, and recovering the expressed polypeptides.

The vectors may be for example, plasmid, virus or phage vectors provided with an origin of replication, optionally a promoter for the expression of the said polynucleotide and optionally a regulator of the promoter. The vectors may contain one or more selectable marker genes, for example an ampicillin resistance gene in the case of a bacterial plasmid or a neomycin resistance gene for a mammalian vector. Vectors may be used *in vitro,* for example for the production of RNA or used to transfect or transform a host cell. The vector may also be adapted to be used *in vivo*, for example in a method of gene therapy.

A further embodiment of the invention provides host cells transformed or transfected with the vectors for the replication and expression of polynucleotides of the invention. The cells will be chosen to be compatible with the said vector and may for example be bacterial, yeast, insect or mammalian.

### E. Therapeutic uses

The invention is based on the finding that Salmosin can inhibit the function of αvβ3 integrin and strongly inhibit tumour angiogenesis which is essential for cancer cell growth and metastasis without affecting proliferation of normal endothelial cells.

Salmosin and fragments and homologues thereof which inhibit integrin function may therefore be used as antitumour agents. Polynucleotides encoding these polypeptides may also be used inhibit tumour growth and metastasis by gene therapy.

Thus the polypeptides and polynucleotides of the invention may be used as outlined above in compounds for treating tumours in animals or humans. Typically the compounds are formulated for clinical administration by mixing them with a pharmaceutically acceptable carrier or diluent. For example they can be formulated for topical, parenteral, intravenous, intramuscular, subcutaneous, intraocular or transdermal administration. Preferably, the compound is used in an injectable form. Direct injection into the patient's tumour is advantageous because it makes it possible to concentrate the therapeutic effect at the level of the affected tissues. It may therefore be mixed with any vehicle which is pharmaceutically acceptable for an injectable formulation, preferably for a direct injection at the site to be treated. The pharmaceutically carrier or diluent may be, for example, sterile or isotonic solutions.

The dose of compound used may be adjusted according to various parameters, especially according to the compound used, the age, weight and condition of the patient to be treated, the mode of administration used, pathology of the tumour and the required clinical regimen. As a guide, the amount of compound administered by injection is suitably from 0.01 mg/kg to 30 mg/kg, preferably from 0.1 mg/kg to 10 mg/kg.

The routes of administration and dosages described are intended only as a guide since a skilled practitioner will be able to determine readily the optimum route of administration and dosage for any particular patient and condition.

Compounds to be administered may include polypeptides or nucleic acids that encode polypeptides. Nucleic acids may be administered by, for example, lipofection or by viral vectors. For example, the nucleic acid may form part of a viral vector such as an adenovirus. When viral vectors are used, in general the dose administered is between 10⁴ and 10¹⁴ pfu/ml, preferably 10⁶ to 10¹⁰ pfu/ml. The term pfu ("plaque forming unit") corresponds to the infectivity of a virus solution and is determined by infecting an appropriate cell culture and measuring, generally after 48 hours, the number of plaques of infected cells. The techniques for determining the pfu titre of a viral solution are well documented in the literature.

Any cancer types may be treated by these methods, for example leukaemias, and solid tumours such as breast, ovary, lung, colon, pancreas, testes, liver, brain, muscle and bone tumour.

The following examples illustrate the invention:

### Example 1: Isolation of Salmosin from venom

To purify Salmosin from venom of Korean snake, *Agkistrodon halys brevicaudus,* 1ml of venom was suspended with 20mM Tris·HCl-buffer(pH 7.5, "buffer A") to a volume of 10ml, and applied into benzamidine-Sepharose(Pharmacia-LKB, Sweden) column which is equilibrated with buffer A, at a flow rate of 30ml/hr. The unbound fractions of the column were collected and suspended with buffer A, and applied again into DEAE-Toyopearl column (2.5×5.0cm)(Pharmacia-LKB, Sweden) at a flow rate of 60ml/hr. The step elution was carried out at concentrations of 25mM, 50mM, 100mM and 1M NaCl and the active fractions were concentrated with ultrafiltration system(Amicon, U.S.A.). The concentrated material was applied onto TSK-G2000 column(7.5×300mm) (Toyosoda, Japan) and eluted with PBS(phosphate buffered saline). Active fractions were pooled, concentrated and applied onto reverse-phase Vydac C18 column(2.5×300mm) (Vydac, U.S.A.) equilibrated with 0.1% TFA(trifluoroacetic acid) solution. And then, the column was alternately washed with the said TFA solution and 20%(v/v) acetonitrile solution and the protein was eluted with a linear gradient of 20 - 60%(v/v) acetonitrile in the said TFA solution.

### Example 2: Expression and purification of recombinant Salmosin

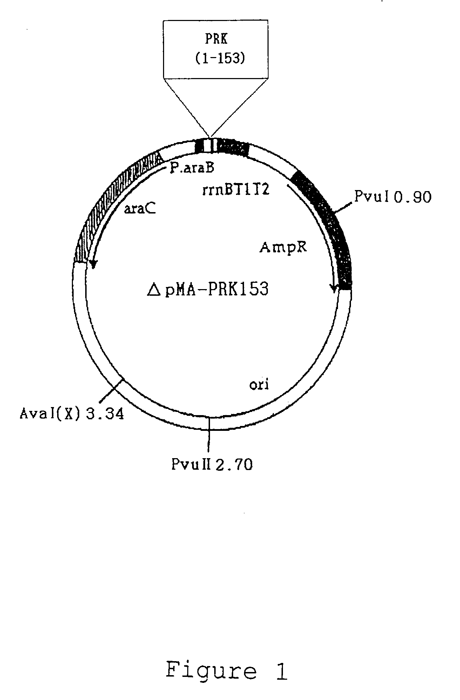

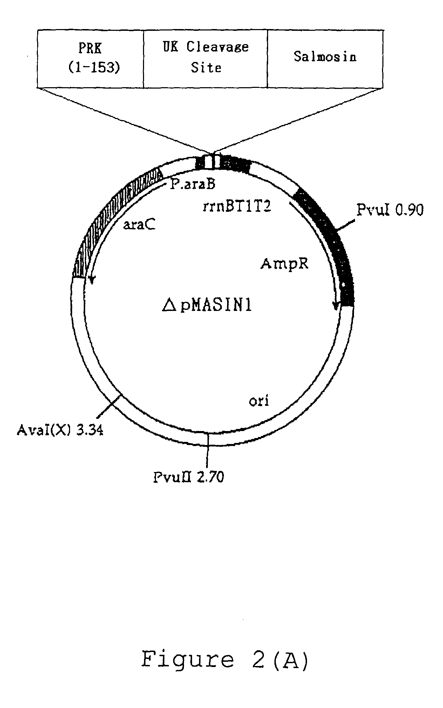

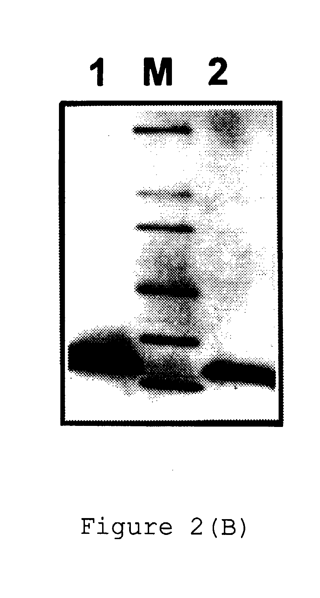

To clone cDNA encoding Salmosin, polymerase chain reaction(PCR) was performed with the venom gland cDNA library of *Agkistrodon halys brevicaudus* as a template. In carrying out PCR, oligo d(T) was employed as 3' primer and the nucleotide sequence(SEQ ID NO: 2) deduced from N-terminal amino acid sequence(-EECDCG-) of Salmosin was employed as 5' primer. PCR product of 290bp was purified by agarose gel electrophoresis and cloned into a vector pCRII (Invitrogen, U.S.A.). The cloned DNA sequence was analyzed(SEQ ID NO: 3) and the amino acid sequence translated from the said DNA sequence was consistent with that of isolated Salmosin. For the expression of recombinant Salmosin, an *E.coli* expression vector, ΔpMA-PRK153 was employed(see: Figure 1). In this vector, the protein of interest is expressed as a fusion protein to the phosphoribulose kinase(PRK) under the control of the araB promoter. For the facilitation of the subcloning, the DNA fragment generated by PCR was modified by introducing BamHI and XhoI site. Urokinase cleavage site was also introduced between the coding sequences of phosphoribulose kinase and Salmosin and an expression vector thus prepared was designated as ΔpMASIN1(see: Figure 2A) . In Figure 2A, P araB represents araB promoter, PRK(1-153) represents the protein comprising amino acid sequence from 1 to 153 and UK represents the cleavage site of urokinase. The said expression vector was transformed in the *E.coli* strain MC1061. The transformants were grown in 1L of 2X YT medium to an absorbance of 0.3 at a wavelength of 600nm, induced with 1% arabinose(w/v) and incubated at 37 °C for 18hr. Salmosin was expressed as an insoluble inclusion body and the inclusion body was resuspended in a pellet wash solution(0.5% Triton X-100, lmM EDTA, lmM DTT) and centrifuged at 12,000rpm for 20min to remove contaminated *E.coli* proteins. After washing 3 more times, the pellet was solubilized in 20ml of 8M urea solution(8M urea, 20mM Tris·HCl, pH 7.8, 20 µM DTT) and incubated at 4°C for 24hr. After centrifugation, the clear supernatant was dialyzed against 4L of dialysis buffer (80mM NaCl, 20mM Tris·HCl, pH 7.8, 0.03% SDS) at 4°C. The refolded fusion protein was then cleaved with urokinase at a ratio of 400mg of fusion protein and 1 U of urokinase at room temperature for 30min. 10mg of cleaved protein was loaded onto DEAE-Toyopearl column equilibrated with 20mM Tris·HCl(pH 8.0) buffer and eluted with the same buffer containing 50mM NaCl. Active fractions were pooled, concentrated and applied onto TSK-G200 HPLC column(2.15 X 30cm) and eluted with PBS. Subsequently, to remove the inappropriately cleaved Salmosin, active fractions were applied onto semi-preparative C18 HPLC column(1.0 X 20cm) and eluted with a linear gradient of 20-40%(v/v) acetonitrile in 0.1% TFA solution.

### Example 3: Comparative assay of recombinant Salmosin and isolated Salmosin

To compare physicochemical natures and biological activities of Salmosin prepared in Examples 1 and 2 described above, N-terminal sequencing, the platelet aggregation assay, and SDS-gel electrophoresis were carried out, respectively. The results showed that N-terminal sequence of recombinant Salmosin is initiated with the sequence of EAGEEC, which is consistent with that of isolated Salmosin and the suppressive acitivity of platelet aggregation is also identical. And, the result of SDS-PAGE indicated that the size of the recombianant Salmosin is exactly the same as that of the isolated salmosin(see: Figure 2B). In Figure 2B, lane 1 is the recombinant Salmosin, lane M is size marker, and lane 2 is the isolated Salmosin. Accordingly, it was clearly demonstrated that the recombinant Salmosin is correctly refolded and has the same biological activity as the isolated Salmosin.

### Example 4: Inhibition of metastasis by Salmosin

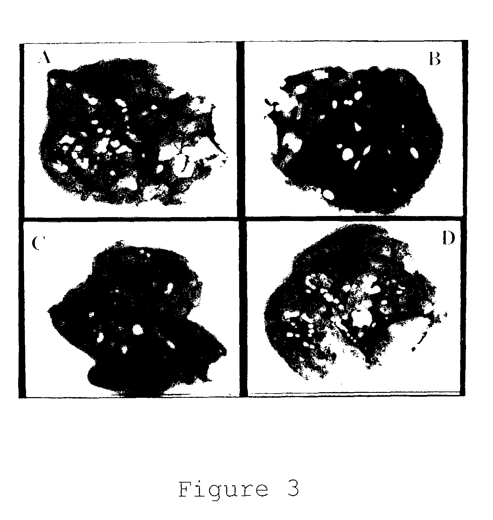

To investigate the inhibitory effect of Salmosin on tumor metastasis *in vivo,* Salmosin was coinjected with B16 melanoma cells into C57BL/6 mouse(Charles river, Japan). B16 melanoma cells were detached with 0.02% EDTA and resuspended gently to 7.5×10⁵/ml in RPMI-1640 medium without serum. Salmosin(250, 500, 1,250µg/kg mouse) was then mixed with the cells to prepare single-cell suspension containing the indicated amount of Salmosin or PBS, and the single-cell suspension of 200µl aliquots were injected into the lateral tail veins of mice. 14 days later, the mice were sacrificed and the number of lung melanoma colonies was counted by dissecting microscope. As a result, it was detected that metastatic colonies were dramatically reduced when compared with the PBS-treated control group in the lungs of C57BL/6 mice(see: Table 1). As shown in Table 1, the inhibition of colony formation by Salmosin was in dose-dependent manner; lower dose(250µg/kg mouse) of Salmosin was able to inhibit the colony formation in the lung, but few colonies were detectable when salmosin was administered with higher dose. These findings are consistent with histochemical analysis(see: Figure 3: A, normal lung; B, PBS treatment; C, 250µg Salmosin/kg treatment; D, 1,250µg Salmosin/kg treatment)

**Table 1:**

| Inhibition of metastasis by the treatment of Salmosin | | | |
|---|---|---|---|
| Salmosin (µg/kg mouse) | Number of mouse | Average number of lung tumor colony | Percentage of inhibition (%) |
| 0 | 8 | 144 ± 40 | 0 |
| 250 | 7 | 49 ± 22 | 66 |
| 500 | 7 | 3 ± 2 | 98 |
| 1,250 | 6 | 1 ± 1 | 99 |

Further, it was demonstrated that the inhibition of metastasis by Salmosin is not caused by cytotoxicity, based on the experimental fact that incubation of the B16 melanoma cells *in vitro* with Salmosin did not affect their subsequent proliferation rate.

These experimental evidences strongly suggested that Salmosin inhibits tumor metastasis by acting as an antagonist of integrin receptors on the surface of tumor cells, based on the suppressive effect of Salmosin on adhesion and invasion of B16 melanoma cell to ECM.

### Example 5: Inhibition of angiogenesis by Salmosin

### Example 5-1: BCE(bovine capillary endothelial) cell proliferation assay

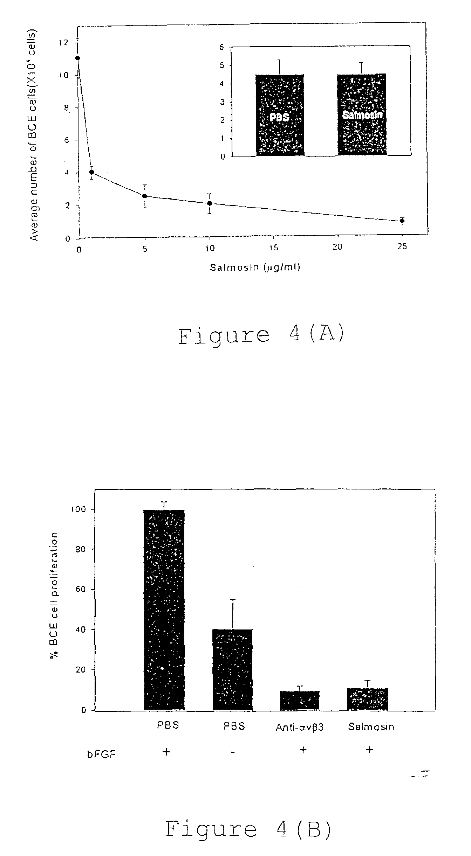

The suppressive effect of Salmosin on angiogensis was examined by employing BCE cell proliferation assay system. Primary culture of BCE cell from bovine adrenal was prepared, and then the cells were maintained in Dulbeco's minimum essential medium("DMEM") containing 3ng/ml of bFGF supplemented with 10% fetal calf serum. The said BCE cells were plated onto gelatinized 24-well culture plates and incubated at 37°C, 5% CO₂ for 24hr. After incubation, the medium was replaced with DMEM containing 5% fetal calf serum, and Salmosin was added to the medium. After 20 min of incubation, bFGF(1ng/ml) dissolved in the said medium was treated to the cells and after 72hr, cells were detached with trypsin and the number of cells was counted. As a result, it was found that Salmosin is able to inhibit the proliferation of BCE cells stimulated by bFGF in a dose-dependent manner. Half-maximal inhibition was observed with Salmosin concentration of 0.1-0.2µg/ml corresponding to 13-27nM(see: Figure 4A). In addition, the bFGF-stimulated BCE cells undergo remarkable morphological change into spherical shape by the treatment of Salmosin. However, it was observed that the cell proliferation is not inhibited in the absence of bFGF when the cells were treated with 20µg/ml Salmosin which was required for maximal inhibition of bFGF-induced proliferation(see: graph in box of Figure 4A). On the other hand, it was examined that anti-αvβ3 monoclonal antibody effectively inhibits bFGF-stimulated proliferation of BCE cells(see: Figure 4B).

### Example 5-2: CAM(chick chorioallantoic membrane) assay

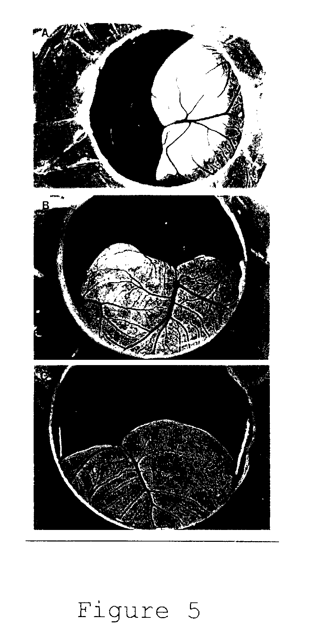

The suppressive effect of Salmosin on tumor-induced angiogenesis *in vivo,* was examined by CAM (chick chorioallantoic membrane) assay: Three-day-old fertilized eggs were carefully cracked and sealed with transparent tapes. For 10 days of incubation at 37°C with 60% humidity, the said fertilized eggs were developed into embryo. After that, 6ng of bFGF was implanted on the CAM of individual embryo to induce neovascularization. After 24hr of incubation, 5µg of Salmosin, 5µg of anti-αvβ3 moniclonal antibody(positive control) or PBS(phosphate buffered saline, negative control) was treated on the CAM of individual embryo. And after 72hr, blood vessels on CAM were observed under the stereomicroscope(see: Figures 5A, 5B and 5C). It was observed that: there was no visible change in the neovascularization when PBS was treated(see: Figure 5A), while it was inhibited when Salmosin or anti-αvβ3 moniclonal antibody was treated(see: Figures 5B and 5C). Especially, it was found that Salmosin breaks new blood vessels which are induced by bFGF, indicating that Salmosin inhibits tumor-induced angiogenesis, without affecting the preexisting blood vessels or the angiogenesis that is a normal physiological phenomenon.

### Example 6: Inhibition of BCE cell adhesion by Salmosin

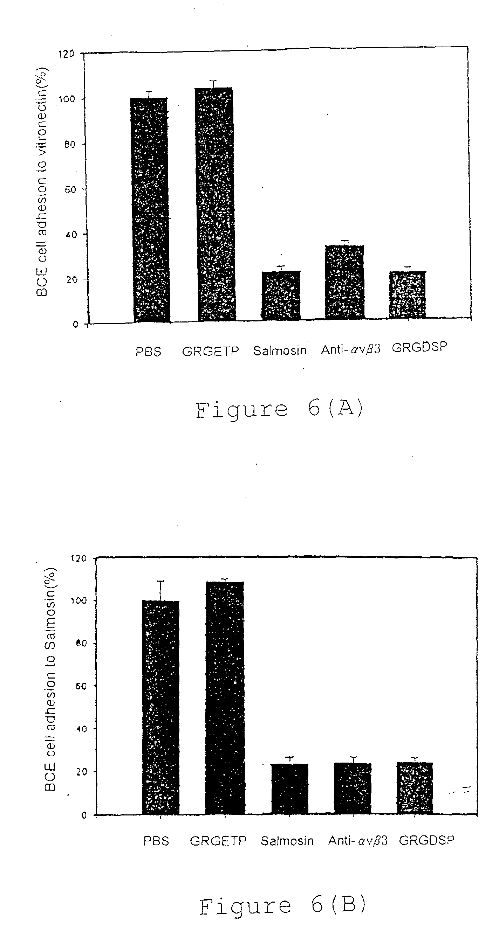

To investigate whether the BCE cell proliferation is inhibited by direct-binding of Salmosin to αvβ3 integrin which is a vitronectin receptor on the surface of BCE cell, the capability of Salmosin which inhibits BCE cell adhesion to vitronectin was examined: 96-well microplates were coated with Salmosin(1µg/well) and vitronectin(0.5µg/well) in PBS at 4°C for 16hr. The said microplates were washed and incubated for lhr with 10mg/ml of heat-denatured bovine serum albumin to block remaining protein binding sites, and washed with PBS before use. BCE cells were detached with trypsin-EDTA, washed three times in PBS, and resuspended in serum-free DMEM. 5×10⁴ of cells were preincubated with Salmosin, anti-αvβ3 monoclonal antibody, synthetic RGD peptide(GRGDSP) or synthetic RGE peptide(GRGETP) for 20min and aliquoted to each well of the said plate, then further incubated at 37°C for lhr in 5% CO₂, 95% air. Unbound cells were removed by washing with PBS, then the cells which bound to the plate were fixed and stained with Coomassie Blue. Absorbance at 540nm of the individual well was measured to determine the relative cell number. As a result, it was found that: adhesion of BCE cell to vitronectin can be highly suppressed by preincubating the bFGF-stimulated cells with Salmosin(see: Figure 6A); anti-αvβ3 monoclonal antibody strongly inhibits the cell adhesion to Salmosin(see: Figure 6B); and, synthetic RGD peptide(GRGDSP) is also able to prevent the cells from adhering to either vitronectin or Salmosin(see: Figures 6A and 6B). These results clearly demonstrated that Salmosin binds to αvβ3 integrin on BCE cells and thereby blocks the integrin-mediated cell adhesion.

### Example 7: Inhibition of metastatic tumor growth by Salmosin

It has been reported that disintegrins suppress the colony formation of lung tumor by inhibiting the attachment of tumor cells to endothelium. However, it is not clear whether the disintegrins inhibit the growth of lung metastatic tumor or not. To investigate the suppressive effect of Salmosin on metastatic tumor growth, 1.5×10⁵ of Lewis lung carcinoma cells obtained from ATCC(Rockville, U.S.A.) were injected into the lateral tail veins of 8-week-old male C57BL/6 mice, and metastatic colonies were developed in mice. 4 days later, Salmosin was administered intravenously to the mice once a day with a dose of 1.25mg/kg/day; 4 weeks later, mice were sacrificed and lungs were removed; the number of lung tumor colony was counted under the dissecting microscope. and there was no recognizable symptom of toxicity in any mouse tested. As a result, it was demostrated that Salmosin remarkably inhibits the metastatic tumor growth(see: Table 2). In this regard, the suppressive effect of Salmosin on the metastatic tumor growth could be explained by binding of Salmosin to αvβ3 integrin which is essential for tumor angiogenesis.

**Table 2:**

| Growth inhibition of pulmonary metastatic Lewis lung carcinoma by Salmosin treatment | | | |
|---|---|---|---|
| Salmosin (mg/kg mouse) | Number of mouse | Average number of lung tumor colony | Inhibition (%) |
| o | 4 | 15 ± 6 | 0 |
| 4 | 4 | 0.8 ± 0.7 | 93 |

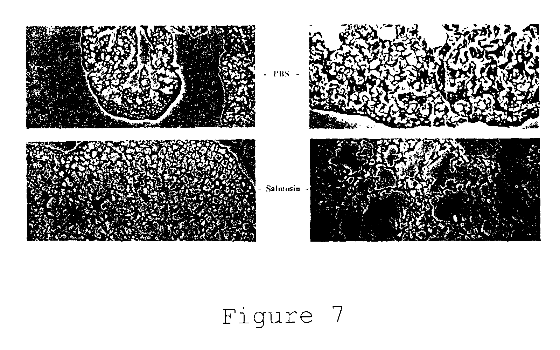

Further, histochemical analysis was carried out to investigate a suppressive effect of Salmosin on the pulmonary metastatic tumor. The said lung tissue was fixed for 4hr in Bouin's soution and embedded in paraffin in accordance with the standard procedure. Sections of 4 µm thick were permeabilized with trypsin at 37°C for 10 min and washed with PBS. The said sections were stained with hematoxylin and eosin, and then mounted. It was observed that few of tumor colonies were detected in the lungs of Salmosin-treated animal(see: Figure 7), which is quite different from those of PBS-treated control mice. Figure 7 shows photographs of ×400 magnification(left) and ×200 magnification(right), respectively, suggesting that the suppressive effect of Salmosin on metastatic tumor growth is closely related to the suppression of angiogenesis which is necessary for secondary tumor growth.

### Example 8: Inhibition of solid tumor growth by Salmosin

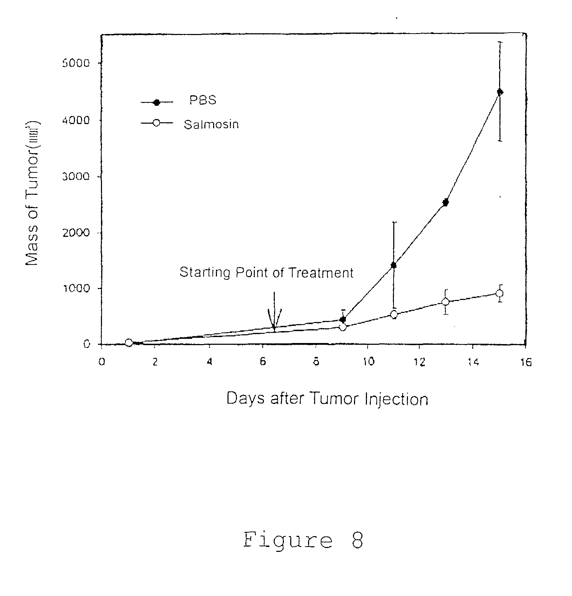

To examine the role of Salmosin in the growth of solid tumor, Lewis lung carcinoma cells(1×10⁶) were injected subcutaneously into dorsal midline of C57BL/6 mice and grown to a mass of at least 100-200mm³. After the mice were randomized into two groups, one group received injections of Salmosin(lOmg/kg mouse) in PBS via subcutaneous injection at a site distant from the tumor once daily and the other group as a control received injections of PBS alone. The size of the tumors in all groups were measured at the same time everyday and the experiments were terminated when the control mice began to die. As a result, it was observed that the growth of solid tumor is strongly inhibited by the treatment of Samosin(see: Figure 8)

### Administration and effective dose

Though the effective dose of Salmosin is variable depending on age, body weight of patient and progression of disease, it is preferable to administer parenterally 0.5 to 10mg/kg/day in a single dose, and which may be individualized by experience of the skilled in the art.

### Acute toxicity test

To examine the acute toxicity of Salmosin, Salmosin was injected into male C57BL/6 mouse subcutaneously and the deads were counted to determine LD₅₀ for 7days. As a result, LD₅₀ is determined as about 1,000mg/kg, indicating that the anti-tumor agent comprising Salmosin is sufficiently safe within the range of effective dose.

The pharmaceutical composition of the present invention which comprises an active ingredient of Salmosin and pharmaceutically acceptable carrier may be administrated as an injection formula. The injection formula may further comprise isotonic aqueous solution or suspension, and the pharmaceutically acceptable carrier covers preservatives, stabilizers, wetting agents, emulsifiers, salts for changing osmotic pressure or buffers.

While the present invention has been shown and described with reference to the particular embodiments employing melanoma cells and Lewis lung carcinoma cells as tested tumor cells, the anti-tumor agent of the invention may be effective for various tumors such as skin cancer, laryngeal cancer, uterine cancer, colon cancer, lung cancer and bone marrow cancer.

As clearly illustrated and demonstrated as above, the present invention provides an anti-tumor agent comprising an active ingredient of Samosin. In accordance with the present invention, it was demonstrated that Salmosin is a novel disintegrin which blocks the function of αvβ3 integrin and strongly inhibits tumor angiogenesis, tumor metastasis as well as growth of solid tumor. Salmosin does not exhibit cytotoxicity within an effective dose range where tumor growth is efficiently suppressed without any untoward effect on preexisting blood vessels and normal angiogenesis. Accordingly, Salmosin can be applied for the development of potent anti-tumor drugs which are effective for various types of cancers.

## Claims

1. A polypeptide which comprises the sequence of SEQ ID NO: 1, a polypeptide substantially homologous thereto, or a fragment of the polypeptide of, or homologue of, SEQ ID NO: 1 for use in a method of treatment of the human or animal body.

2. Use of a polypeptide as defined in claim 1 in the manufacture of a medicament for anti-tumor therapy.

3. Use of a polypeptide according to claim 2 in the manufacture of a medicament for the inhibition or reduction of tumor cell angiogenesis.

4. Use of a polypeptide according to claim 2 in the manufacture of a medicament for the inhibition of metastatic tumor formation.

5. Use of a polypeptide according to claim 2 in the manufacture of a medicament for the inhibition or reduction of metastatic tumor growth.

6. Use of a polypeptide according to any one of claims 2 to 5 for use in the manufacture of a medicament for the treatment of skin cancer, laryngeal cancer, uterine cancer, colon cancer, lung cancer or bone marrow cancer.

7. A polynucleotide selected from:
(a) a polynucleotide comprising the nucleotide sequence of SEQ ID NO: 3 or the complement thereof
(b) a polynucleotide comprising a nucleotide sequence capable of hybridizing to the nucleotide sequence defined in (a)
(c) a polynucleotide comprising a nucleotide sequence which is degenerate as a result of the genetic code to a polynucleotide as defined in (a) or (b).

8. A polynucleotide according to claim 7 which encodes a polypeptide as defined in claim 1.

9. An expression vector comprising a polynucleotide as defined in claim 7 or 8 operably linked to regulatory sequences capable of directing expression of said polynucleotides in a host cell.

10. A pharmaceutical composition comprising a polypeptide as defined in claim 1, a polynucleotide according to claim 7 or 8 or a vector according to claim 9 and a pharmaceutically acceptable carrier.


### Annex to the description

SEQUENCE LISTING

<110>    KIM, Doo-Sik

<120>    Anti-Tumor Agent Comprising Salmosin as an Active Ingredient

<130>    N77164 GCW

<150>    KR 98-23778
<151>    1998-06-23

<150>    KR 99-20579
<151>    1999-06-04

<160>    5

<210>    1
<211>    73
<212>    PRT
<213>    Agkistrodon halys brevicaudus

<400>    1
Glu Ala Gly Glu Glu Cys Asp Cys Gly Ser Pro Gly Asn Pro Cys Cys
1               5                   10                  15

Asp Ala Ala Thr Cys Lys Leu Arg Gln Gly Ala Gln Cys Ala Glu Gly
            20                  25                  30

Leu Cys Cys Asp Gln Cys Arg Phe Met Lys Glu Gly Thr Ile Cys Arg
        35                  40                  45

Arg Ala Arg Gly Asp Asp Leu Asp Asp Tyr Cys Asn Gly Ile Ser Ala
    50                  55                  60

Gly Cys Pro Arg Asn Pro Phe His Ala
65                  70

<210>    2
<211>    20
<212>    DNA
<213>    Artificial Sequence

<220>
<223>    single stranded oligonucleotide

<400>    2
ggngargart gygaytgygg

<210>    3
<211>    222
<212>    DNA

<213>    Agkistrodon halys brevicaudus

<400>    3
gaggccggag aagaatgtga ctgtggctct cctggaaatc cgtgctgtga tgctgcaacc      60

tgtaaactga gacaaggagc acagtgtgca gaaggactgt gctgtgacca gtgcagattt     120

atgaaagaag gaacaatatg ccggagagca aggggtgatg acctggatga ttactgcaat     180

ggcatatctg ctggctgtcc cagaaatccc ttccatgcct aa                        222


<210>    4
<211>    6
<212>    PRT
<213>    Artificial Sequence

<400>    1
Gly Arg Gly Asp Ser Pro
1               5



<210>    5
<211>    6
<212>    PRT
<213>    Artificial Sequence

<400>    1
Gly Arg Gly Glu Thr Pro
1               5